## Europäisches Patentamt

## European Patent Office

## Office européen des brevets

(11) Publication number: **0 235 923**
**B1**

# (12) EUROPEAN PATENT SPECIFICATION

(45) Date of publication of patent specification: **09.01.91**

(21) Application number: **87300702.5**

(22) Date of filing: **28.01.87**

(51) Int. Cl.⁵: **C 12 N 15/49,** C 12 P 21/00,
G 01 N 33/68

(54) **Sor gene product from human t-cell lymphotropic virus III.**

(30) Priority: **31.01.86 US 824783**

(43) Date of publication of application:
**09.09.87 Bulletin 87/37**

(45) Publication of the grant of the patent:
**09.01.91 Bulletin 91/02**

(84) Designated Contracting States:
**AT BE CH DE FR GB IT LI LU NL SE**

(56) References cited:
**EP-A-0 173 529**

**NATURE, vol. 313, no. 6000, January 24, 1985
(New York, London); L. RATNER et
al.:"Complete nucleotide sequence of the AIDS
virus, HTLV-III, pp. 277-284**

**NATURE, vol. 315, May 9, 1985 (New York,
London); N.T. CHANG et al.:"An HTLV-III
peptide produced by recombinant DNA is
immunoreactive with sera from patients with
AIDS", pp. 151-154**

(73) Proprietor: **THE UNITED STATES OF AMERICA
as represented by the Secretary, United States
Department of Commerce
National Technical Information Service, Office of
Government Inventions and Patents, 5285 Port
Royal Road
Springfield, Virginia 22161 (US)**

(72) Inventor: **Papas, Takis S.
10700 Tulip Lane
Potomac Maryland 21701 (US)**
Inventor: **Kan, Nancy Chen
9 Polaris Building
Hershey Pennsylvania 17033 (US)**
Inventor: **Lautenberger, James Allen
4492 Willowtree Drive
Middletown Maryland 21769 (US)**
Inventor: **Wong-Staal, Flossie
3 Lynn Manor Court
Rockville Maryland 20850 (US)**

(74) Representative: **Daley, Michael John et al
F.J. CLEVELAND & COMPANY 40/43 Chancery
Lane
London, WC2A 1JQ (GB)**

EP 0 235 923 B1

(56) References cited

**NATURE, vol. 312, November 8, 1984 (New York, London); B.H. HAHN et al.:"Molecular cloning and characterization of the HTLV-III virus associated with AIDS", pp. 166-169**

**SCIENCE, vol. 228, no. 4695, April 5, 1985 (Washington D.C.); N.T. CHANG et al.:"Expression in Escherichia coli of Open Reading Frame Gene Segments of HTLV-III", pp. 93-96**

**CELL, vol. 40, no. 1, January 1985 (Cambridge, Mass. USA); S. WAIN-HOBSON et al.:"Nucleotide Sequence of the AIDS Virus, LAV", pp. 9-17**

**Description**

## BACKGROUND OF THE INVENTION

Human T-lymphotropic virus type III (HTLV-III/LAV) is a retrovirus which has been implicated as the causative agent of acquired immunodeficiency syndrome in man (AIDS). The published nucleotide sequences of three isolates of HTLV-III and related viruses predict the characteristic retroviral *gag*, *pol*, and *env* genes as well as two small open reading frames, the *sor* and 3' *orf*. The two small open reading frames are unique to the HTLV-III genome in that they have no equivalents similar in size and location in the genomes of other exogenous retroviruses, including the two other human retroviruses HTLV-I and HTLV-II. The *sor* region is located between the *pol* and *env* genes and can encode a protein of 193 amino acids with an approximate molecular weight of 20 kilodaltons (Kd). Its initiator methionine overlaps with the end of *pol* and its terminator precedes the beginning of *env*. The 440 nucleotides between the *sor* and *env* genes contain additional open reading frames which have the potential to encode small novel proteins. After the *env* gene there is an additional gene called 3' *orf* which extends into the 3' long terminal repeat (LTD). The native gene product of 3' *orf* in HTLV-III infected cells has been identified as a 27 Kd protein and shown to be immunogenic *in vivo*. The present invention is the HTLV-III *sor* gene product corresponding to a 23 Kd protein which is also immunogenic *in vivo*. Antibodies to this *sor* protein and diagnostic test kits are also disclosed.

HTLV-III is an exogenous retrovirus which infects mainly the OKT4+ hunan T-cells. Unlike most other non-defective retroviruses, infection with HTLV-III generally leads to cell death *in vitro* and to depletion of T-cells with OKT4 phenotype in patients with AIDS. The smnall open reading frames unique to the HTLV-III genome may contribute to this cytopathic effect by encoding viral proteins which either directly or indirectly alter the cell metabolism. DNA probes derived from the *sor* region have recently been shown to hybridize to RNA species in HTLV-III-infected cells.

## GENERAL DESCRIPTION OF THE INVENTION

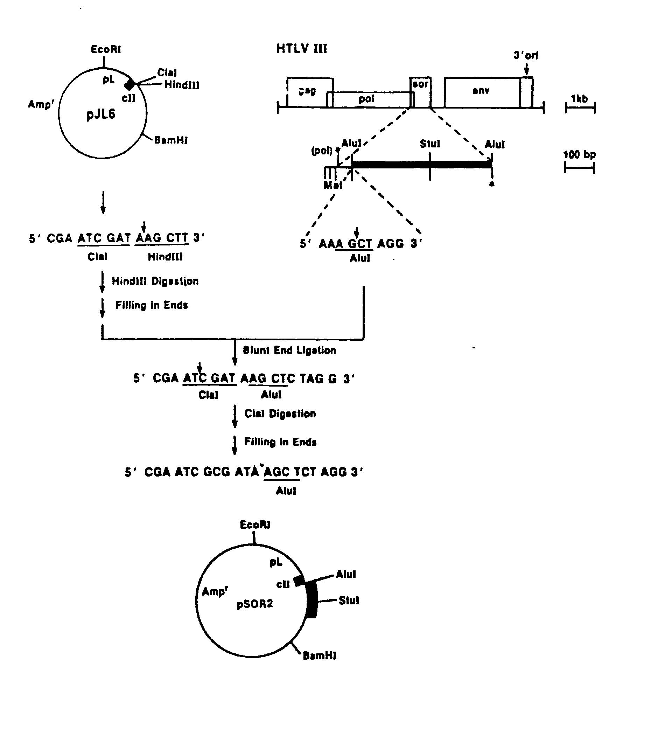

The amino acid sequences which encode the production of the *sor* protein are found in the AluI-AluI fragment of a clone of hunan T-cell lymphotropic virus type III. The nucleotide segment 4724—5201 from an HTLV-III clone such as BH—10 is isolated and placed in a suitable expression vector. The 5' AluI site is located 33 amino acids downstream from the initiator methionine, while the 3' AluI site coincides with the termination codon of the *sor* open reading frame. The AluI fragment is joined to an expression vector, preferably pJL6, at the unique HindIII site as illustrated in The Figure. Plasmid pJL6 provides the initiator methionine of the phage lambda cII gene. The cII-*sor* hybrid gene thus formed, as well as the bacterially synthesized *sor* protein, contains 15 amino acids derived from the cII gene at its amino terminus and the remaining 160 amino acids derived from the HTLV-III *sor* region. The plasmid containing the cII-*sor* hybrid gene may be inserted into E. coli bacteria in order to produce the *sor* protein. This protein may be purified and used in HTLV-III diagnostic assays, or may be used to produce monoclonal antibodies which specifically bind to the *sor* protein amino acid sequences.

## STATEMENT OF DEPOSIT

Plasmid pSOR2 was deposited in the American Type Culture Collection in Rockville, Maryland, on January 31, 1986 under ATCC No. 53457, and will be maintained for a term of thirty (30) years or five (5) years after the last request for such deposit or for the effective life of the patent, whichever is longest. The deposit will be replaced if the culture mutates or becomes nonviable during the term of the deposit.

## DESCRIPTION OF THE FIGURE

The Figure illustrates the construction of a plasmid containing the AluI-AluI fragment from Human T-cell Lymphotropic Virus Type III and the initiator methionine of the lambda cII gene.

## SPECIFIC DESCRIPTION OF THE INVENTION

The AluI-AluI fragment of Human T-cell Lymphotropic Virus Type III (HTLV-III) is derived from the BH—10 clone corresponding to positions 4724—5201. The BH—10 clone is a well known HTLV-III clone described in Hahn et al, *Nature*, Vol. 312, p. 166 (1984). Although the use of clone BH—10 is preferred, the present invention should not be restricted thereby. Other HTLV-III clones which include the AluI-AluI fragment, such as HXB—2, HXB—3, or BH—5/BH—8, are incorporated within the scope of the invention. Furthermore, the amino acid sequences noted above correspond to the BH—10 clone, the AluI-AluI fragment from other clones may be defined by slightly different amino acid sequence numbers, (for a description of the numbering method used in this invention, see Ratner et al, *Nature*, Vol. 313, p. 277 (1985)).

The AluI-AluI fragment defined above encodes 160 amino acids out of a maximun 193 determined by the *sor* open reading frame, thus covering at least 83% of the HTLV-III *sor* gene. The AluI fragment is joined to an expression vector pJL6, containing a unique HindIII site located 13 amino acids downstream from the initiator methionine of the lambda cII gene. Although vector pJL6 is the preferred vector of the present invention, other vectors may be used if they contain a cII gene initiator methionine.

The process of inserting the AluI fragment into expression vector pJL6 involves well known procedures. As is shown in the Figure, pJL6 DNA is cleaved with HindIII and the cohesive ends are converted to blunt ends by E. coli polymerase. The AluI fragment from an HTLV-III clone — the 5' AluI site located 33 amino acids downstream from the putative initiator methionine, while the 3' AluI site coincides with the termination codon of the *sor* open reading frame — is then blunt end-ligated to the linearized pJL6 DNA by T4 DNA ligase. The phage lambda cII gene contained in pJL6 provides thirteen amino acids at the N-terminus including the initiator methionine for the *sor*-containing protein. A plasmid, termed pSOR1, was shown to contain the HTLV-III *sor* sequence cloned in the correct orientation by observing the unique StuI site near the middle of the AluI fragment. To adjust the frame of the translation of the inserted sequence, pSOR1 DNA is cleaved with ClaI, treated with E. coli polymerase to fill the cohesive ends, and religated by T4 ligase. The resulting plasmid, termed pSOR2, is shown to have the expected reading frame by observing a new NruI site replacing the ClaI site, and by direct sequencing of the vector-insert junction.

To verify that the proper sequences are present in pSOR2, double-stranded pSOR2 DNA is denatured, renatured in the presence of a synthetic oligonucleotide derived from the end-terminus of the cII gene, and sequenced according to known methods of sequence analysis. The DNA sequence of about 100 nucleotides demonstrates that pSOR2 contains an open reading frame starting at the cII gene in the vector that extends in frame into the *sor* open reading frame. The bacterially synthesized *sor* protein, with an estimated molecular weight of 18 Kd, thus contains 15 amino acids derived from the cII gene at its amino terminus and the remaining 160 amino acids derived from the HTLV-III *sor* region.

The cII-*sor* hybrid gene in pSOR2 is under the transcriptional control of the lamba *pL* promoter located immediately upstream from the cII gene. In order to produce *sor* protein, pSOR2 is placed in E. coli MZ1 (containing a temperature sensitive lambda repressor). When total E. coli proteins are metabolically labelled with [$^{35}$S]methionine or [$^{35}$S]cysteine, a new protein of 20 Kd is produced in the temperature-induced MZ1 cells containing pSOR2. This protein is not observed in uninduced cells, nor in induced cells containing the vector alone, or with pSOR1 (with an out-of-frame *sor* region). Coomassie blue staining shows that the *sor* protein constitutes approximately 10% of total E. coli proteins. The *sor* protein is purified by successive extractions with salts and detergents as described by Krippel et al (*PNAS*, Vol. 81, p. 6988 [1984]).

In order to produce *sor*-specific antibodies, the bacterially synthesized *sor* protein is purified using reverse-phase HPLC by the Procedure of Samuel et al, *Gene Anal. Tech.*, Vol. 2, p. 60 (1985). Two prominent protein bands coelute from the C$_{18}$ column. These two bands constituted over 95% of the protein present. A rabbit is immunized by three 100 ug inoculations of this reverse-phase HPLC purified *sor* protein. Western blot assay demonstrates that the rabbit antiserum reacts strongly with both species of the *sor* protein purified by the cation-exchange HPLC column. The rabbit antiserum is then used to identify the native *sor* protein in HTLV-III-infected H9 cells. Cells are metabolically labeled and the cell extract is immunoprecipitated with the rabbit antiserum. Only the immune serum, but not the pre-immune serum, recognized a 23 Kd protein in the HTLV-III-infected cells. The serum of a hemophiliac individual asymptomatic of AIDS, but seropositive for HTLV-III envelope antigens also reacts with a protein from the same cell extract which comigrated with the 23 Kd protein. A protein of the same size in HTLV-III infected cells is also detected with the sera from HTLV-III seropositive people and identified to be the native *sor* gene product by partial amino acid sequencing.

## EXAMPLES

### Example 1

Partially purified *sor* protein was used in a Western blot assay to test its immunoreactivity against human sera. Seventy nine sera were obtained from patients seropositive from other HTLV-III antigens (either p24 and/or gp41) and 16 sera from normal individuals with no detectable antibodies against the HTLV-III *gag* and *env* proteins. The group of 79 seropositive patients included: 29 individuals "at risk", such as homosexuals, IV drug users and relatives of AIDS patients; 22 patients with AIDS-related complex (ARC); and 28 patients with AIDS. Antibodies were detected that reacted with the *sor* protein in some people of all the groups that were analyzed. Approximately half of the sera from AIDS and healthy "at risk" patients reacted with the bacterially synthesized *sor* protein while only one third of the sera from patients with ARC were positive. The results are summarized in Table 1.

TABLE 1
Frequency of human natural antibodies against
bacterially synthesized *sor* protein

| Clinical Status | No. Tested | No. Positive | % Positive |
|---|---|---|---|
| Healthy donors | 16 | 4 | 25 |
| Healthy at risk | 29* | 12 | 41 |
| ARC | 22* | 7 | 32 |
| AIDS | 28* | 15 | 53 |

\* All these sera were also positive for antibodies to other viral antigens (either p24 and/or gp41).

Our results indicate that the *sor* protein is immunogenic *in vivo* and that there is a significantly higher prevalence of antibodies against the HTLV-III *sor* protein in the infected patients than that in normal people.

## Example 2
*Analysis of the bacterially synthesized sor* protein and its immunoreactivity with human sera.

(A) plasmid pSOR2 was grown in DC646 cells and used later to transform MZ1 that contains a temperature sensitive repressor (cl857). MZ1 cells containing the vector pJL6, or the plasmid pSOR2, or the plasmid pSOR1, were grown in supplemented M56 media at 32°C to an optical density at 590 of about 0.4. An aliquot of 50 ul was metabolically labelled with [$^{35}$S]methionine at 42°C for 30 seconds, and total *E. coli* proteins were visualized on a 10% SDS-polyacrylaminde gel by autoradiography. The size of the new protein is consistent with the predicted molecular weight of the HTLV-III Alul fragment (160 amino acids) plus the N-terminus of the CII gene (15 amino acids). The results obtained with [$^{35}$S]cysteine were identical to the ones shown above.

(B) *E. coli* MZ1 cells containing either the vector pJL6, or the plasmid pSOR2, were induced at 42°C for 1 hour, and total *E. coli* proteins were analyzed on a 10% SDS-polyacrylaminde gel stained with Coomassie blue.

(C) The cII-*sor* hybrid protein was extracted from two-liters culture of MZ1 containing pSOR2 as described in the specific disclosure. The protein in the potassium thiocyanate pellet fraction (1 mg) was solubilized in 50mM Tris, pH 7.5, 2mM DTT, 7M urea and an aliquot was analyzed on a 12.5% SDS-polyacrylamide gel stained with Coomassie blue. The two *sor* protein species are probably the result of internal initiation in the *sor* region. Approximately 400 ug of the protein was applied to a cation-exchange Waters Protein-Pak SP—5PW HPLC column equilibrated with 20mM sodium phosphate, pH 7.0 and 7 M urea. The two species of the *sor* protein were eluted in different fractions with a linear 25-min gradient of 0—80% of 0.75M NaCl in the equilibration buffer. Aliquots of the peak fractions were analyzed on a 12.5% SDA-polyacrylamide gel stained with Coomassie blue.

(D) The partially purified *sor* protein fraction shown in section (C), was electrophoresed on a 15% SDS-polyacrylamide gel, transferred to a nitrocellulose filter, and reacted with human sera (1:100 dilution) by the Western blot assay. The antigen-antibody complexes were detected using [$^{125}$I] protein A.

## Example 3
*Characterization of rabbit antiserum to HTLV-III sor* protein.

Rabbit antiserum to HPLC-purified *sor* protein was used in Western immunoblot (A) and immunoprecipitation (B) analyses. Bacterially synthesized *sor* protein, extracted by salts and detergents as described by Krippl *et al.* and purified by reverse phase HPLC using a C$^{18}$ column as described by Samuel *et al.*, was used to elicit antibodies. The primary immunization was with 100 ug of the protein described above followed by two 100 ug boosts 10 and 20 days later. The antiserum was used to react with the purified *sor* protein electrophoresed on a 10% SDS-polyacrylamide gel, and immobilized on a nitrocellulose filter by the Western blot assay. Immune complexes were detected using [$^{125}$I] protein A. HTLV-III infected H9 cells were labeled with equal activities of [$^{35}$S]methionine and [$^{35}$S]cysteine, and lysed. The cell extract was immumoprecipitated with: the rabbit pre-immune serum; the rabbit antiserum as described in (A); the serum from a hemophiliac patient; and the serum from another hemophiliac patient, followed by electrophoresis om a 10% SDS-polyacrylamide gel. The HTLV-III envelope gp160 and the envelope gp160 and gp120 and *gag* p55 and p24 of the autoradiogram indicates that the patients have been exposed to the virus.

## TEST KITS
Plasmid pSOR2 may be transfected into an immortalizing cell line such as H9 and the cells, antigens, or proteins produced from those cells may be incorporated as an antigen in test kits. Such a kit includes, but is not limited to, the plasmid(s) or cells transfected with a plasmid of the present invention bound to a solid support. In general, the present inventiom includes antibody reagent kits suitable for use in the detection of *sor* gene product in human samples. Furthermore, the present invention is useful in Western blot kits, immunoblot kits, or ELISA plates in order to determine levels of antibodies against *sor* in human sera, including sera from AC/AIDS patients.

Immortalized cell lines containing the plasmid(s) of the present invention, or segments thereof, may be used in an enzyme linked immunosorbent assay (ELISA). A diagnostic test kit for the detection of AIDS-specific antibodies comprises a compartmented enclosure containing a plurality of wells or plates (employing a sorbent or porous surface) which are coated prior to use with an antigen or protein derived from the cells transfected with the plasmid or DNA sequences obtained from the plasmid. The plasmid or DNA sequences boumd to the porous surface is then incubated with a test sample of human blood. After removal of the sample, known methods of conducting an ELISA assay are employed: coating the wells with normal goat serum and peroxidase-labeled goat anti-human immunoglobulin and a color change indicator (such as orthophenylene diamine and hydrogen peroxide in a phosphate citrate buffer).

Other forms of a test kit may also be constructed. For example, competition radioimmunoassay or Dot Blot assays include a compartmented container with a cover, a sorbent or porous surface such as a nitrocellulose sheet, surfactants, pH modifiers, bovine serum albunin, human Fab, dilute normal goat serum, and $^{125}$I-labeled animal antihuman immunoglobulin.

## Claims

1. A process for the production of human T-cell lymphotropic virus type III *sor* protein, chaacterised by inserting an Alu I-Alu I fragment obtained from an HTLV-III clone into a plasmid vector and transforming said vector into a bacterial cell.

2. The process of claim 1, further characterised in that said AluI fragment is an HTLV-III clone BH—10 segment corresponding substantially to nucleotide sequence 4724—5201.

3. Plasmid pSOR2, deposited in the American Type Culture Collection under accession number 53457, and containing an AluI-AluI fragment from human T-cell lymphotropic virus.

4. A process for the production of antibodies which specifically bind to the *sor* gene of Human T-cell Lymphotropic Virus Type III, characterised by immunizing a mammalian host with purified *sor* protein, and isolating and purifying antibodies which specifically bind to said *sor* protein.

5. A test kit comprising a compartmented enclosure, characterized by containing plasmids or cells transfected with plasmid pSOR2 deposited in American Type Culture Collection under Accession No. 53457, bound to a solid support.

## Patentansprüche

1. Verfahren zur Herstellung von human-T-Zellen-lymphotropem Virus Typ III-Sor-Protein, gekennzeichnet durch Einsetzen eines von einem HTLV-III-Klon erhaltenen AluI-AluI-Fragments in einen Plasmidvektor und Transformieren dieses Vektors in eine bakterielle Zelle.

2. Verfahren nach Anspruch 1, weiter dadurch gekennzeichnet, daß das genannte AluI-Fragment ein Segment des HTLV-III-Klons BH—10 entsprechend im wesentlichen der Nukleotidsequenz 4724—5201 ist.

3. Plasmid pSOR2, niedergelegt in der American Type Culture Collection unter Zugriffsnummer 53457, und ein AluI-AluI-Fragment aus dem huamn-T-Zellen-lymphotropen Virus enthaltend.

4. Verfahren zur Herstellung von Antikörpern, die sich spezifisch an das sor-Gen des human-T-Zellen-lymphotropen Virus Typ III binden, gekennzeichnet durch Immunisieren eines Säugetier-Wirts mit gereinigtem sor-Protein, und Isolieren und Reinigen von Antikörpern, die sich spezifisch an dieses sor-Protein biden.

5. Testausrüstung mit einem in Kammern unterteilten Behälter, dadurch gekennzeichnet, daß er Plasmide oder Zellen enthält, die mit an einen festen Träger gebundenem, in der American Type Culture Collection unter Zugriffsnummer 53457 niedergelegten Plasmid pSOR2 transfektiert sind.

## Revendications

1. Procédé de production d'une protéine *sor* de virus lymphotropique de type III de cellule T humaine, caractérisé en ce qu'on insère un fragment AluI-AluI obtenu à partir d'un clone d'HTLV-III dans un vecteur de plasmide et en ce qu'on transforme ledit vecteur dans une cellule bactérienne.

2. Procédé de la revendication 1, caractérisé en outre en ce que ledit fragment AluI est un segment du clone BH—10 d'HTLV-III correspondant pour l'essentiel à la séquence de nucléotide 4724—5201.

3. Plasmide pSOR2, déposé à l'American Type Culture Collection sous le n° 53457, et contenant un fragment AluI-AluI provenant d'un virus lymphotropique de cellule T humaine.

4. Procédé de production d'anticorps qui se lient de manière spécifique au gène *sor* du virus lymphotropique type III de cellule T humaine, caractérisé en ce qu'on immunise un hôte mammifère avec la protéine *sor* purifiée, et en ce qu'on isole et en ce qu'on purifie les anticorps qui se lient spécifiquement à ladite protéine *sor*.

5. Nécessaire expérimental comprenant un réceptacle compartimenté, caractérisé en ce qu'il contient des plasmides ou cellules transfectés avec le plasmide pSOR2 déposé à l'American Type Culture Collection sous le n° 53457, liés à un support solide.